# EUROPEAN PATENT APPLICATION

(11) **EP 4 759 341 A1**
(43) Date of publication of application: **17.06.2026**
(21) Application number: 24220111.9
(22) Date of filing: 16.12.2024
(51) Int. Cl.: A61M 5/00

(54) **HOLDING TOOL FOR IDENTIFYING SYRINGE SIZE**

(71) Applicant: Becton, Dickinson and Company, Franklin Lakes, NJ 07417 (US)
(72) Inventor: SILLARD, Yann, 38560 Champ sur Drac (FR); FREMON, Benoit, 38410 Saint Martin d'Uriage (FR); HELL, Julien, 38700 La Tronche (FR)
(74) Representative: Germain Maureau

(57) **Abstract**

Provided herein is a syringe holder syringe holder for enabling measurement of a syringe barrel having a cylindrical main body and a flange positioned at a proximal end of the cylindrical main body. The syringe holder includes at least one rack comprising a top plate including a plurality of top holding apertures, a bottom plate including a plurality of bottom holding apertures, and a plurality of vertical members extending between the top and bottom plates, to space the top plate apart from the bottom plate. Each top holding aperture is aligned with a respective bottom holding aperture, with respective vertically aligned top and bottom holding apertures forming a receptacle configured to receive a syringe barrel therein. A diameter of the top holding aperture of each receptacle in the rack is incrementally different from the diameters of the top holding apertures of the other receptacles.

## Description

### BACKGROUND OF THE INVENTION

### Field of the Invention

The present disclosure relates generally to syringes and, more particularly, to a holding tool used for identifying the size of a syringe barrel.

### Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity of a syringe when the stopper is inserted into the syringe.

Components of the syringes as described above often need to be transported and assembled during or after manufacturing. For instance, syringe barrels may be transported from a manufacturing site to a site at which the barrels may be filled with a medication, with stoppers then inserted into the barrels to seal the medication within the barrel. The filling and stoppering of the syringe barrels are an automated process that is performed by a fill and finish machine.

As part of the assembly of syringe components as described above, is recognized that prefilled syringes must be manufactured to high quality standards. As one example, tolerances and measurements of an inner diameter of the syringe barrel must be precise in order that the stopper fits tightly within the barrel of the syringe, so as to maintain container closure integrity of the syringe when the stopper is inserted into the barrel. As another example, tolerances and measurements of an outer diameter of the syringe barrel must be precise in order that the syringe barrel fits properly within a holding device - which in some embodiments may comprise a holding plate or "nest" within which the syringe barrel is retained during a fill and finish operation, or in other embodiments may comprise an endless screw or starwheel. With such considerations in mind, it is desirable to ensure the geometric dimension of the barrel inner and/or outer diameter at a defined height, so as to ensure proper fitting of a stopper therein that will maintain container closure integrity of the syringe and to ensure proper fit of the syringe barrel within in a nest. The barrel outer diameter is typically measured manually using, for example, a ruler, calibers, etc. However, it is recognized that manually measuring the barrel outer diameter is time consuming and subject to human error.

Accordingly, a need exists in the art for a means for quickly and accurately measuring components of a syringe, and in particular a diameter of a syringe barrel at a defined height.

### SUMMARY OF THE INVENTION

Provided herein is a syringe holder for enabling a syringe barrel having a cylindrical main body and a flange positioned at a proximal end of the cylindrical main body. The syringe holder includes at least one rack comprising a top plate including a plurality of top holding apertures, a bottom plate including a plurality of bottom holding apertures, and a plurality of vertical members extending between the top and bottom plates, to space the top plate apart from the bottom plate. Each top holding aperture of the plurality of top holding apertures is aligned with a respective bottom holding aperture of the plurality of bottom holding apertures, with respective vertically aligned top and bottom holding apertures forming a receptacle configured to receive a syringe barrel therein. A diameter of the top holding aperture of each receptacle in the rack is incrementally different from the diameters of the top holding apertures of the other receptacles.

In certain configurations, the diameter of the top holding aperture of a respective receptacle differs from the diameter of the top holding aperture of an adjacent receptacle by a predetermined incremental amount.

In certain configurations, the diameter of the top holding apertures of a respective receptacle differs from the diameter of the top holding apertures of an adjacent receptacle by 0.05 mm.

In certain configurations, the plurality of top holding apertures includes a nominal holding aperture whose diameter theoretically matches a designed outer diameter of the syringe barrel.

In certain configurations, the diameters of the plurality of top holding apertures ranges from +/- 0.3 mm in diameter from the nominal holding aperture diameter.

In certain configurations, the plurality of top holding apertures in the top plate and the plurality of bottom holding apertures in the bottom plate are each in a linear arrangement.

In certain configurations, the plurality of top holding apertures comprises five top holding apertures and the plurality of bottom holding apertures comprises five bottom holding apertures.

In certain configurations, the plurality of vertical members is arranged such that a vertical member is provided at each of opposing ends of the rack and such that a vertical member is provided between each adjacent pair of receptacles.

In certain configurations, each of the plurality of vertical members comprises a notch formed therein that enable a focused measurement of an angle of the syringe barrel at a defined height thereof.

In certain configurations, the at least one rack comprises a plurality of racks, with the plurality of racks arranged in parallel.

In certain configurations, the syringe holder is a modular holder, where the plurality of racks may be selectively assembled and disassembled from one another to form the modular syringe holder.

In certain configurations, the syringe holder further includes a screen positioned between each adjacent pair of racks, with the screen formed of a dark colored material, such that a contrast is provided between the syringe holder and the syringe barrel that eases observing of the positioning of the flange relative to the syringe holder.

In certain configurations, the diameters of the top holding apertures in each rack are incrementally different from the diameters of the top holding apertures in the other racks,

Also provided herein is a method for measuring an outer diameter of a syringe barrel. The method includes attempting to place the syringe barrel within a nominal receptacle of the syringe holder. When the syringe barrel does not fit within the nominal receptacle, the method includes attempting to place the syringe barrel within the adjacent receptacle whose diameter is incrementally larger than the nominal receptacle and continuing to attempt placement of the syringe barrel within incrementally larger receptacles until a receptacle is found having a diameter that accommodates positioning of the syringe barrel therein in a desired configuration. When the syringe barrel fits within the nominal receptacle, the method includes attempting to place the syringe barrel within the adjacent receptacle whose diameter is incrementally smaller than the nominal receptacle and continuing to attempt placement of the syringe barrel within incrementally smaller receptacles until a receptacle is found having a diameter that accommodates positioning of the syringe barrel therein in a desired configuration.

In certain configurations, positioning of the syringe barrel in a receptacle in the desired configuration comprises a distal-facing surface of the flange of the syringe barrel being brought into complete contact with the top plate.

In certain configurations, the method further includes positioning the syringe holder onto a platform of a microscope and observing contact between the flange and the top plate to measure a perpendicularity of the barrel to the top plate.

In certain configurations, the nominal receptacle comprises a receptacle whose diameter theoretically matches a designed outer diameter of the syringe barrel.

### BRIEF DESCRIPTION OF THE DRAWINGS

FIG. 1 is a perspective view of a syringe barrel with which embodiments of the disclosure may be implemented;
FIG. 2 is a perspective view of a syringe holding tool, according to a non-limiting embodiment described herein; and
FIG. 3 illustrates the syringe holding tool of FIG. 2 positioned on a microscope, for visual inspection of a syringe barrel retained in the syringe holding tool, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

Aspects and embodiments of the disclosure are directed to a syringe holding tool used for identifying the size of a syringe barrel, for purposes of quality control when manufacturing the syringe barrel and ensuring that critical dimensions of the syringe barrel are within a desired tolerance range. A syringe barrel may be inserted into each of a plurality of receptacles provided in the holding tool, to obtain a measurement of an outer diameter of the syringe barrel at a defined height on the syringe barrel.

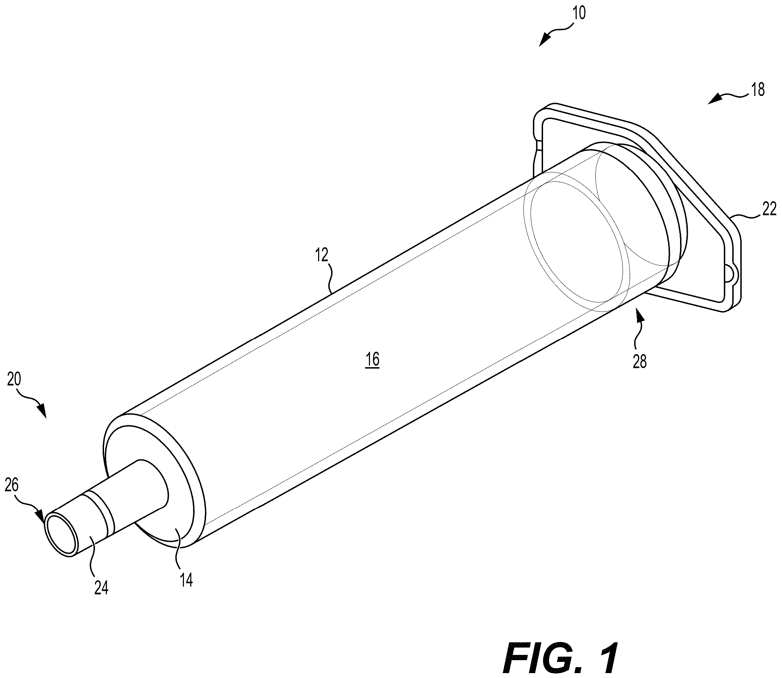

Referring to FIG. 1, shown is a non-limiting embodiment of a syringe barrel 10 with which aspects or embodiments of the disclosure may be implemented. The syringe barrel 10 may be part of a syringe that also includes a plunger assembly (not shown), with the plunger assembly being axially movable within the syringe barrel 10 to an advanced position to facilitate administering of an injectable fluid (e.g., medication) to a patient, for example. As known in the art, the plunger assembly may include a plunger rod having a stopper attached to a distal end thereof, with stopper formed from a material that is different from the material of the plunger rod, such as rubber or another elastomeric material, and that is capable of forming a tight seal with the syringe barrel 10 as it is advanced therethrough.

The syringe barrel 10 is formed of a cylindrical main body 12 and an end wall 14 that collectively define a chamber 16 for retaining fluid therein. The syringe barrel 10 includes an open proximal end 18 configured to receive a plunger assembly therein and a distal end 20 at which end wall 14 is positioned. The proximal end 18 of the syringe barrel 10 may include a flange 22 to facilitate handling and positioning of the syringe 10 and to maintain the relative position of the syringe barrel 10 with respect to the plunger assembly during medication administration. In one embodiment, the distal end 20 of syringe barrel 10 may include a tip 24 that extends distally outward from the end wall 14 and defines an opening or lumen 26 in fluid communication with the chamber 16. In some embodiments, and as illustrated in FIG. 1, the tip 24 is configured as a luer connection or luer fitting that may mate with a corresponding luer connection or luer fitting of a vascular access device (when administering fluid from the syringe barrel) or a fluid source (when aspirating fluid into the syringe barrel). It can be appreciated that, in other embodiments, tip 24 may be provided as a hub within which a needle is secured (i.e., a staked needle syringe) and extends out distally therefrom.

As previously described, it is known in the art that prefilled syringes must be manufactured to high quality standards, so that critical dimensions of the syringe barrel - such as the outer diameter of the syringe barrel - are within a desired tolerance range. The controlling of the outer diameter of the syringe barrel at a defined height thereof ensures that the syringe barrel fits properly into an associated holding device - such as a chimney of a nest within which the syringe barrel is retained during processing (i.e., during a fill and finish processing operation), or fits properly with an endless screw or starwheel, as other non-limiting examples.

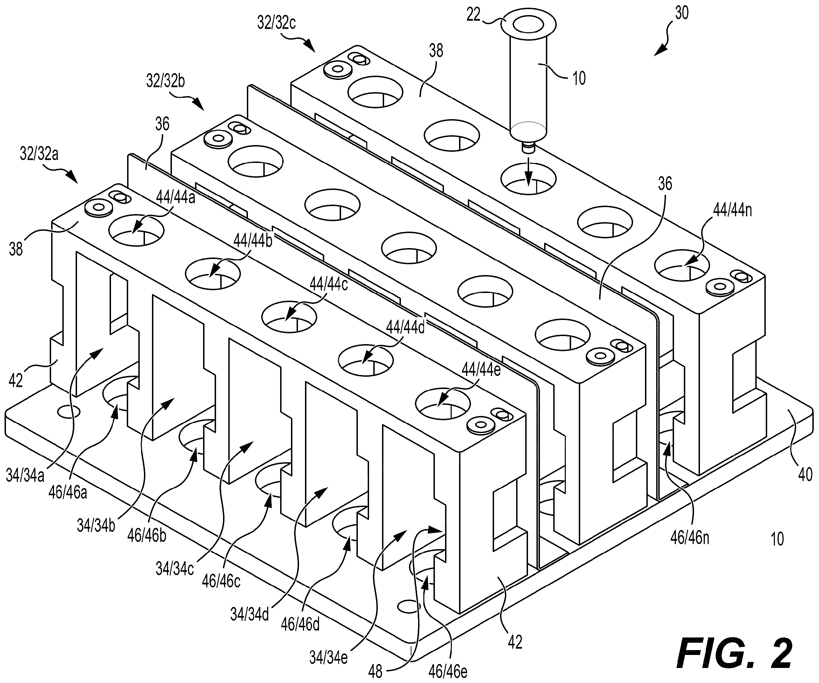

Referring now to FIG. 2, a syringe holding tool 30 (hereafter "syringe holder 30") is illustrated according to an aspect of the disclosure. The syringe holder 30 may comprises a plurality of racks 32, with each of the racks defining a plurality of receptacles 34 arranged in a linear fashion that are configured to receive a syringe barrel therein, such as the syringe barrel 10 illustrated in FIG. 1. According to embodiments, the receptacles 34 may be designed to accommodate the barrel of any of a number of different syringe sizes/volumes, such as 1 mL syringes, 5 mL syringes, or 10 mL syringes, as non-limiting examples. In the illustrated embodiment, the syringe holder 30 is formed of an arrangement of three racks 32, arranged in parallel. However, it is recognized that syringe holder 30 could include a greater or lesser number of racks 32. Additionally, while each rack 32 of the syringe holder 30 is shown as including an arrangement of five (5) receptacles 34 therein (i.e., receptacles 34a, 34b, 34c, 34d, 34e), it is recognized that each rack 32 could include a greater or lesser number of receptacles 34. Where the syringe holder 30 includes multiple racks 32 arranged in parallel, a screen 36 may be positioned between each adjacent pair of racks 32. Each screen 36 may be formed of a dark colored material, such that a contrast is provided between the syringe holder 30 and the syringe barrel 10 that eases observing of the positioning of the flange 22 relative to the syringe holder 30, as explained in further detail below.

In some embodiments, the syringe holder 30 may be of a single construction such as an injected molded piece of polycarbonate material or the like, such that the racks 32 are collectively formed as a single, integral syringe holder. In other embodiments, the syringe holder 30 may be configured as a modular holder, where the racks 32 may be selectively assembled and disassembled from one another to form a modular syringe holder 30 of desired size. In some embodiments, the racks 32 may be machined from stainless steel and polyethetherketone (PEEK).

As shown in FIG. 2, each rack 32 of the syringe holder 30 includes a top plate 38, a bottom plate 40, and a plurality of vertical members 42 extending between the top and bottom plates 38, 40 to space the top plate 38 apart from the bottom plate 40. The top plate 38 and bottom plate 40 each include a plurality of container holding apertures 44, 46 That is, the top plate 38 includes a plurality of top holding apertures 44 and the bottom plate 40 includes a plurality of bottom holding apertures 46, with each top holding aperture in the top plate 38 vertically aligned with a respective bottom holding aperture in the bottom plate 40, such that each respective pairing of a top holding aperture 44 and bottom holding aperture 46 forms a receptacle 34 within which a syringe barrel 10 may be received. In some embodiments, top plate 38 and bottom plate 40 may each include five (5) holding apertures 44, 46 formed therein (such that five receptacles 34 are provided in rack 32), with it recognized that a greater or lesser number of holding apertures 44, 46 could be formed in the top and bottom plates 38, 40.

The vertical members 42 of rack 32 are arranged such that a vertical member 42 is provided at each of opposing ends of the rack 32 and such that a vertical member 42 is provided between each adjacent pair of receptacles 34 (i.e., between each adjacent pair of top holding apertures 44 and each adjacent pair of bottom holding apertures 46). With the vertical members 42 arranged as such, each receptacle 34 of rack 32 is isolated/separated from an adjacent receptacle 34. In some embodiments, each of the plurality of vertical members 42 comprises a notch 48 formed therein that enables a focused measurement of an angle of the syringe barrel 10 at a defined height thereof, as explained in more detail below.

According to aspects of the disclosure, each of the receptacles 34 provided in the rack 32 is configured to have a different sizing. Specifically, the top holding aperture 44 of each receptacle 34 may have a different diameter than the top holding apertures 44 of the other receptacles 34, while the bottom holding apertures 46 may all have a constant diameter big enough to let the syringe barrel 10 pass therethrough. According to aspects of the disclosure, the diameter of the top holding aperture 44 of each receptacle 34 is incrementally adjusted as compared to the diameters of the top holding apertures 44 of the other receptacles 34. According to one non-limiting embodiment, the diameter of each top holding aperture 44 differs from its adjacent top holding aperture(s) 44 by 0.01 to 0.5mm although it is recognized that a different incremental adjustment may be used. Thus, as one example, an arrangement of five holding apertures 44a, 44b, 44c, 44d, 44e may each differ by 0.05 mm.

For a syringe holder 30 including multiple racks 32, such as a first rack 32a, second rack 32b, and third rack 32c, the diameters of the holding apertures 44 in each rack could be incrementally different from the diameters of the holding apertures 44 in the other racks. Thus, the range in diameter between a smallest holding aperture 44a and a largest holding aperture 44n may be further expanded. Ideally, a minimum range of +/- 0.3 mm in diameter from a nominal holding aperture diameter is provided by the syringe holder 30, to accommodate a wide range in variance of syringe barrel outer diameters.

As a non-limiting example, the first rack 32a may have a center top holding aperture 44c that is nominal ("0"), with top holding apertures 44b, 44a having diameters that are +0.05 mm and +0.1 mm as compared to the nominal, respectively, and the top holding apertures 44c, 44d having diameters that are -0.05 mm and -0.1 mm as compared to the nominal, respectively. The second rack 32b may have a center top holding aperture 44 that is nominal ("0"), with other top holding apertures 44 having diameters that are +0.2 mm, +0.15 mm, -0.15, and -0.2 mm as compared to the nominal. The third rack 32c may have a center top holding aperture 44 that is nominal ("0"), with other top holding apertures 44 having diameters that are +0.3 mm, +0.25 mm, -0.25, and -0.3 mm as compared to the nominal.

According to aspects of the disclosure, the syringe holder 30 may be used to test or measure an outer diameter of a manufactured syringe barrel 10 (selected from a batch/lot of manufactured syringe barrels 10), in order to verify that the syringe barrel 10 meets desired geometric tolerances at a defined height of the syringe barrel 10, such as at a neck 28 of the syringe barrel 10 that is immediately distal to the flange 22 (see FIG. 1). A process for accurately measuring the outer diameter of the syringe barrel 10 at a defined height thereof is described in further detail here below.

In order to measure a syringe barrel 10, a user may attempt to place the syringe barrel 10 within a nominal receptacle 34 of the syringe holder 30 - i.e., a receptacle 34 whose diameter should theoretically match the designed outer diameter of the syringe barrel 10 (at neck 28).

In a first scenario, where the syringe barrel 10 does not fit within the nominal receptacle 34, then the outer diameter of the syringe barrel 10 is determined to be larger than the diameter of the chosen receptacle 34. The user may thus attempt to place the syringe barrel 10 within the adjacent receptacle 34 whose diameter is incrementally larger than the prior receptacle 34, to see if the syringe barrel 10 fits within this receptacle 34. The user may continue this process of attempting placement of the syringe barrel 10 within incrementally larger receptacles 34 until a receptacle 34 is found having a diameter that accommodates positioning of the syringe barrel 10 therein. An appropriately sized receptacle may be considered to be found when the syringe barrel 10 may be fully placed into the receptacle 34 such that the flange 22 of the syringe barrel 10 is brought into complete contact with the top plate 38 of a rack 32.

In a second scenario, the syringe barrel 10 fits within the nominal receptacle 34. The user may then attempt to place the syringe barrel 10 within the adjacent receptacle 34 whose diameter is incrementally smaller than the prior receptacle 34, to see if the syringe barrel 10 fits within this receptacle 34. The user may continue this process of attempting placement of the syringe barrel 10 within incrementally smaller receptacles 34 until a receptacle 34 is found having a diameter that appropriately accommodates positioning of the syringe barrel 10 therein. An appropriately sized receptacle may be considered to be found when the syringe barrel 10 may be fully placed into the receptacle 34 such that the flange 22 of the syringe barrel 10 is brought into complete contact with the top plate 38 of a rack 32.

Via the above-described process, an outer diameter of a manufactured syringe barrel 10 may be accurately at a defined height of the syringe barrel 10 (e.g., at neck 28). With an outer diameter of the syringe barrel 10 accurately measured, complications encountered as part of an automated fill and finish operation may be reduced/eliminated, such as proper fitting of the syringe barrel 10 in a holding plate or nest (or endless screw or starwheel).

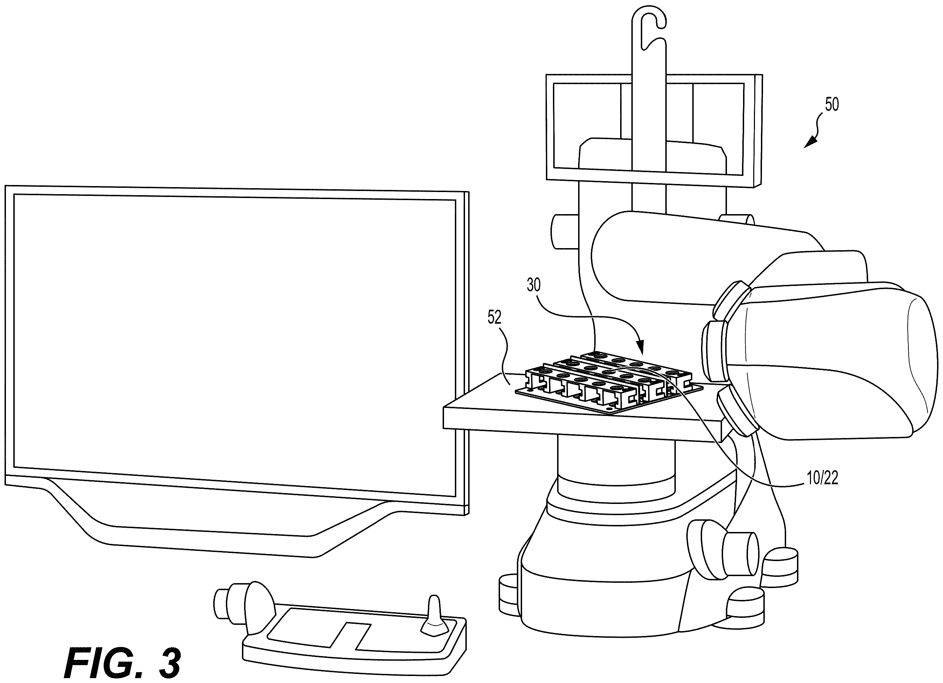

In some embodiments of the invention, the syringe holder 30 may be further configured to be compatible for use with an imaging device or system that may be used to further inspect the tested/measured syringe barrel. As a non-limiting example, and as shown in FIG. 3, the syringe holder 30 may be sized and configured for use with a microscope 50 (e.g., a Keyence microscope). With the syringe barrel 10 received within a selected receptacle 34 where the flange 22 of the syringe barrel 10 is brought into complete contact with the top plate 38 of rack 32, the syringe holder 30 is placed on a platform 52 of the microscope 50, and the microscope 50 is used to observe the contact between the flange 22 and the top plate 38. As part of this observation, the perpendicularity of the flange 22 to the top plate 38 can be measured, to verify that the flange 22 is in complete contact with the top plate 38 and that the appropriately sized receptacle has been selected (for accurately determining the outer diameter of the syringe barrel 10). In some embodiments, screens 36 of the syringe holder 30 may be formed of a dark colored material, such that a contrast is provided between the syringe holder 30 and syringe barrel 10 that eases viewing of the positioning of the flange 22 relative to the syringe holder 30. In some embodiments, the notches 48 formed in the vertical members 42 enable a focused measurement of an angle of the syringe barrel 10 at the defined height thereof via microscope 50.

Beneficially, embodiments of the invention thus provide a syringe holder used for identifying the size of a syringe barrel, for purposes of quality control when manufacturing the syringe barrel and ensuring that critical dimensions of the syringe barrel are within a desired tolerance range. A syringe barrel may be inserted into each of a plurality of receptacles provided in the holding tool, to obtain a measurement of an outer diameter of the syringe barrel at a defined height on the syringe barrel. Via insertion of the syringe barrel into one or more of the receptacles of the syringe holder (until an appropriately sized receptacle is found), a user may quickly determine the outer diameter of the syringe barrel, with such determination of the outer diameter being performed with improved accuracy and confidence. The syringe holder can be used in a variety of environments and on any planar surface, with the syringe holder also compatible for use with an imaging device or system (e.g., microscope) that further inspects a fit between the syringe barrel and the selected receptacle.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A syringe holder for enabling testing or measurement of a syringe barrel having a cylindrical main body and a flange positioned at a proximal end of the cylindrical main body, the syringe holder comprising:
at least one rack comprising:
a top plate including a plurality of top holding apertures;
a bottom plate including a plurality of bottom holding apertures; and
a plurality of vertical members extending between the top and bottom plates, to space the top plate apart from the bottom plate;
wherein each top holding aperture of the plurality of top holding apertures is aligned with a respective bottom holding aperture of the plurality of bottom holding apertures, with respective vertically aligned top and bottom holding apertures forming a receptacle configured to receive a syringe barrel therein; and
wherein a diameter of the top holding aperture of each receptacle in the rack is incrementally different from the diameters of the top holding apertures of the other receptacles.

2. The syringe holder of claim 1, wherein the diameter of the top holding aperture of a respective receptacle differs from the diameter of the top holding aperture of an adjacent receptacle by a predetermined incremental amount.

3. The syringe holder of claim 2, wherein the diameter of the top holding aperture of a respective receptacle differs from the diameter of the top holding aperture of an adjacent receptacle by 0.01 to 0.5mm.

4. The syringe holder of claim 3, wherein the plurality of top holding apertures includes a nominal holding aperture whose diameter theoretically matches a designed outer diameter of the syringe barrel.

5. The syringe holder of claim 3, wherein the diameters of the plurality of top holding apertures ranges from +/- 0.3 mm in diameter from the nominal holding aperture diameter.

6. The syringe holder of any of claims 1-5, wherein the plurality of top holding apertures in the top plate and the plurality of bottom holding apertures in the bottom plate are each in a linear arrangement.

7. The syringe holder of any of claims 1-6, wherein the plurality of top holding apertures comprises five top holding apertures and the plurality of bottom holding apertures comprises five bottom holding apertures.

8. The syringe holder of any of claims 1-7, wherein the plurality of vertical members is arranged such that a vertical member is provided at each of opposing ends of the rack and such that a vertical member is provided between each adjacent pair of receptacles.

9. The syringe holder of any of claims 1-8, wherein each of the plurality of vertical members comprises a notch formed therein that enable a focused measurement of an angle of the syringe barrel at a defined height thereof.

10. The syringe holder of any of claims 1-9, wherein the at least one rack comprises a plurality of racks, with the plurality of racks arranged in parallel.

11. The syringe holder of claim 10, wherein the syringe holder is a modular holder, where the plurality of racks may be selectively assembled and disassembled from one another to form the modular syringe holder.

12. The syringe holder of claim 10 or claim 11, further comprising a screen positioned between each adjacent pair of racks, with the screen formed of a dark colored material, such that a contrast is provided between the syringe holder and the syringe barrel that eases observing of the positioning of the flange relative to the syringe holder.

13. The syringe holder of any of claims 10-12, wherein the diameters of the top holding apertures in each rack are incrementally different from the diameters of the top holding apertures in the other racks.

14. A method for measuring an outer diameter of a syringe barrel using the syringe holder of claim 1, the method comprising:
attempting to place the syringe barrel within a nominal receptacle of the syringe holder;
when the syringe barrel does not fit within the nominal receptacle:
attempting to place the syringe barrel within the adjacent receptacle whose diameter is incrementally larger than the nominal receptacle; and
continuing to attempt placement of the syringe barrel within incrementally larger receptacles until a receptacle is found having a diameter that accommodates positioning of the syringe barrel therein in a desired configuration; or
when the syringe barrel fits within the nominal receptacle:
attempting to place the syringe barrel within the adjacent receptacle whose diameter is incrementally smaller than the nominal receptacle; and
continuing to attempt placement of the syringe barrel within incrementally smaller receptacles until a receptacle is found having a diameter that accommodates positioning of the syringe barrel therein in a desired configuration.

15. The method of claim 14, wherein positioning of the syringe barrel in a receptacle in the desired configuration comprises a distal-facing surface of the flange of the syringe barrel being brought into complete contact with the top plate.

16. The method of claim 15, further comprising:
positioning the syringe holder onto a platform of a microscope; and
observing contact between the flange and the top plate to measure a perpendicularity of the barrel to the top plate.

17. The method of any of claims 14-16, wherein the nominal receptacle comprises a receptacle whose diameter theoretically matches a designed outer diameter of the syringe barrel.
